# EUROPEAN PATENT APPLICATION

(11) **EP 4 151 167 A1**
(43) Date of publication of application: **22.03.2023**
(21) Application number: 21197416.7
(22) Date of filing: 17.09.2021
(51) Int. Cl.: A61B 18/02, A61B 18/12, A61B 34/00, A61B 18/14, A61B 18/00, A61M 5/168

(54) **ASSISTANCE OF BALLOON ABLATION**

(71) Applicant: Koninklijke Philips N.V., 5656 AG Eindhoven (NL)
(72) Inventor: OREN, Eitan, 5656 AE Eindhoven (NL); KEINAN, Ronen, 5656 AE Eindhoven (NL); GLASSNER, Lior, 5656 AE Eindhoven (NL)
(74) Representative: Philips Intellectual Property & Standards

(57) **Abstract**

A mechanism for aiding a balloon ablation therapy procedure, and in particular, to provide information useful for establishing how successfully a balloon has occluded an anatomical cavity. A plurality of electrodes (positioned upstream or distally of the ablation balloon) each provide a signal responsive to the presence and/or absence of a dielectric medium injected into the anatomical cavity. A combined signal is generated, to which the signals provided by the electrodes only contribute if they meet some predetermined criterion/criteria.

## Description

### TECHNICAL FIELD

The present disclosure relates to the field of assisting balloon ablation procedures.

### BACKGROUND

Atrial fibrillation (AF) is an abnormal heart rhythm characterized by rapid and irregular beating of the atria, and may be associated with heart palpitations, fainting, lightheadedness, shortness of breath, or chest pain. The disease is associated with an increased risk of heart failure, dementia, and stroke. AF may be caused by electrical pulses generated by secondary pacers at the ostium of the pulmonary veins. Accordingly, one way of treating AF is by pulmonary vein isolation, which can include ablating the inner wall of the left atrium to form lesions that isolate the ostium of the pulmonary veins from the rest of the left atrium.

An area of increasing interest is the used of balloon-based ablation procedures, such as cryoablation. Compared to traditional ablation procedures (such as those that use a single electrode tip for performing RF ablation and/or ultrasonic ablation), balloon-based ablation procedures have advantages of a shorter procedure time and the ability to create a contiguous lesion around the pulmonary vein ostium in a single shot.

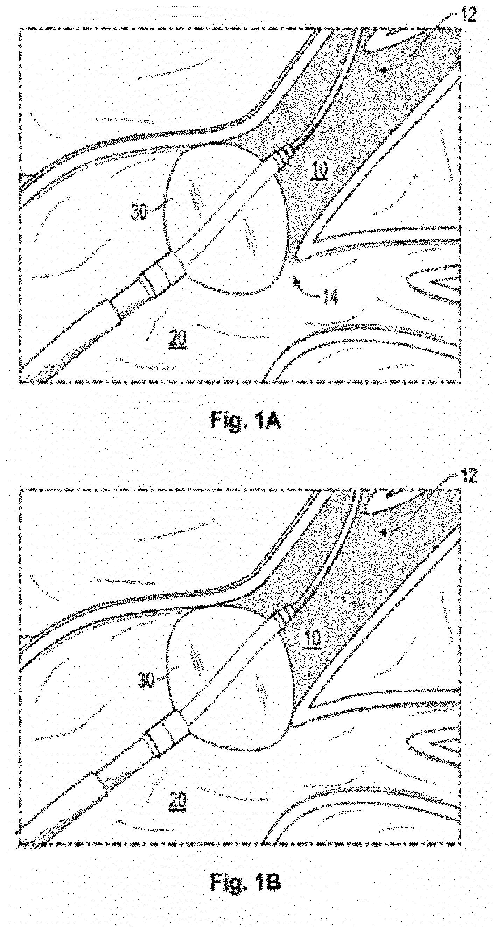

In cryoablation, an inflatable cryoballoon is inflated and cooled to a temperature (e.g., below -65° C) that causes an electrically-isolating lesion or firewall in the tissue. In conventional systems, the cryoballoon is guided to the ablation site, typically the ostium of a pulmonary vein, using fluoroscopy. As illustrated in Figs. 1A and 1B, after having been introduced in the atrium in deflated state, the cryoballoon 30 is inflated, positioned to fully occlude blood flow of the pulmonary vein 10 from the left atrium 20 and subsequently cooled for the ablation. In this way, it can be assured that the lesion formed from the cryoablation will electrically isolate the pulmonary vein from the left atrium. Some challenges with balloon ablation include guiding the ablation balloon (e.g., cryoballoon) to the ablation site, and ensuring that the balloon is placed and oriented to maintain contact with a full circumference of the ostium. If the balloon is misaligned during ablation, the resulting lesion can include one or more gaps which may result in reconduction and recurrence of the arrhythmia thereby requiring a re-do procedure when the AF symptoms return.

Other types of balloon-based ablation procedures are known, such as those that use radio-frequency ablation.

Referring again to Fig. 1A, in conventional methods, angiographic and/or fluoroscopic procedures are used to guide the balloon to the ablation site. Once the inflated balloon 30 is in place, a Fluoroscopic contrast agent 12 also referred to as dye herein is introduced into the pulmonary vein 10 to ascertain pulmonary vein occlusion and detect residual leaks or gaps in the interface between the balloon 30 and the ostium of the pulmonary vein 10 prior to ablating the tissue. When gaps exist, a portion 14 of dye will leak through into the left atrium 20. This leaked portion 14 can be detected by angiography and in particular venography under fluoroscopy, which indicates that the balloon is not yet fully occluding blood flow from the pulmonary vein into the left atrium, and therefore is not optimally positioned to completely isolate the pulmonary vein from the left atrium.

Accordingly, the physician can adjust and reposition the balloon until no residual leaks of the dye are seen, as shown in Fig. IB.

There are some shortcomings to using X ray based angiography or venography to guide balloon ablation procedures. For example, patients and physicians may prefer to avoid x-ray radiation emitted during such procedures. Furthermore, guiding the balloon to the ablation site and checking for leaks in the pulmonary vein-balloon interface may be an imprecise and difficult processes that requires special expertise. It may be contraindicated or unadvisable to use dye for some patients. For example, at least 20% of the population has some type of contraindication for usage of dye, including allergic reactions and kidney failure. Furthermore, due to inherent limitations of dye injection under 2D fluoroscopy, approximately 13% of residual leaks may not be apparent on venography.

There is therefore a desire to make use of alternative methods of identifying occlusion of an anatomical cavity by an ablation balloon.

WO 2013/022853 and US 2017/347896 each disclose balloon ablation systems and methods, in which impedance sensing is used to distinguish between conduction paths through tissue and conduction paths through blood, in order to determine if the balloon has provided the desired occlusion. WO2018/207128 discloses balloon ablation system using catheter electrodes to image the heart and also to provide leakage detection.

WO 2021/001338 A1 proposes an approach in which a dielectric medium is injected into the anatomical cavity that is occluded by an ablation balloon. The electrical signal(s) measured by any electrodes positioned in the occluded anatomical cavity are used to generate occlusion information.

### SUMMARY OF THE INVENTION

The invention is defined by the claims.

According to embodiments of the disclosure, there is proposed a processing system for aiding a balloon ablation therapy procedure that uses an ablation balloon to occlude an anatomical cavity of a subject during the balloon ablation therapy procedure.

The processing system comprises: an input interface configured to receive input data representing a plurality of electrical signals, each electrical signal being measured by a respective one of a plurality of electrodes disposed in the vicinity of the ablation balloon, wherein each one of the plurality of electrical signals is sensitive to the presence of a dielectric medium injected into the anatomical cavity; a processing unit communicatively coupled to the input interface and configured to process the plurality of electrical signals contained in the input data to produce a combined signal, wherein each one or the plurality of electrical signals contributes to the combined signal only when one or more characteristics of said electrical signal meets one or more predetermined criteria; and optionally, an output interface communicatively coupled to the processing unit and configured to provide output data comprising the combined signal.

The present disclosure provides a mechanism for generating a combined signal that provides useful occlusion information for determining at least whether the ablation balloon has occluded the anatomical cavity and/or (if relevant) a potential cause of or reason for the failure to occlude.

Underlying concepts recognize that such a combined signal (from multiple different electric signals) provide a useful overview of the occlusion process, and of the scale and/or rate of any leaks past the ablation balloon. By only allowing electrical signals (measured by different electrodes, which will be spatially separated from one another) to contribute to the combined signal when they meet some predetermined criteria, a reliability and signal-to-noise ratio of the combined signal will be improved. Use of the predetermined criteria also facilitates identification of characteristics that may indicate a potential leak.

The processing system may be configured to control, via the output interface, a user interface to provide a user-perceptible output responsive to the combined signal. For instance, the user interface may be a display (e.g. a screen) and the processing system may control, via the output signal, the screen to provide a visual representation of the combined signal.

The processing unit can comprise a memory storing instructions which when executed by the processing unit cause the processing system to implement the defined steps or those of any of the methods described herein. In some examples of electrophysiology systems including a processing system as defined herein, the execution of the instructions may cause the system to perform the steps. Such electrophysiology systems may comprise dielectric imaging systems or mapping system as known in the art, such as for example KODEX^{™}, CARTO^{™}, EnSite^{™}, Rythmia^{™} or the like.

The instructions of configured processing unit may cause the processing unit to receive the input data and to provide the output data.

In the context of the present disclosure, an electrical signal is sensitive to the presence of a dielectric medium if a discernable and/or measurable (with existing technology) change to the electrical signal results from the introduction of the dielectric medium to the vicinity of the electrical signal.

The one or more characteristics of the electrical signal may comprise an amplitude of the electrical signal and the one or more predetermined criteria may comprise the amplitude of the electrical signal breaching a first predetermined threshold. Breaching a predetermined threshold may indicate that the electrical signal indicates that the measuring electrode is positioned in a location at which no leakage is occurring (at the point of the measurement). By only contributing to the combined signal in these circumstances, the combined signal cam effectively represent an (average or cumulative) non-leakage of the balloon catheter, i.e. a measure of occlusion.

In some examples, the plurality of electrodes are arranged around a hypothetical closed shape in the anatomical cavity. The hypothetical closed shape may, for instance, be an oval or a circle. In some examples, the hypothetical closed shape may be a shape of the boundary of the anatomical cavity. This increases an accuracy of the occlusion information, as any leakage across the entire circumference of the anatomical cavity is more likely to be identified. This approach also facilitates identification of the specific location of a leakage (e.g. by detecting which electrode indicates a leakage is occurring).

The plurality of electrodes may (e.g. in use, during the ablation procedure) be positioned distally of the ablation balloon in the anatomical cavity and/or upstream of a blood flow direction through the anatomical cavity.

The one or more characteristics of the electrical signal may comprise a gradient of the amplitude of the electrical signal and the one or more predetermined criteria comprises the gradient of the amplitude of the electrical signal breaching a second predetermined threshold.

In at least one embodiment, each electrical signal and the combined signal is time dependent; and each electrical signal only contributes to the combined signal, at a particular point in time for the combined signal, when one or more characteristics of the electrical signal at the particular point in time meets one or more predetermined criteria.

In this way, a combined signal representative of leakage and/or occlusion characteristics over a period of time can be generated. This may be in the form of a waveform or other temporal sequence of values. This information aids in identifying the cause and/or reason for a leak (if present) and/or classifying a type of the leak (if present) or occlusion.

In some examples, the step of generating the combined signal may comprise determining a sum of the electrical signals that contribute to the combined signal. The combined signal may comprises the sum of the electrical signals that contribute to the combined signal. As another example, the combined signal may comprise the sum of the electrical signals that contribute to the combined signal divided by the total number of electrical signals (both contributing and non-contributing).

In some examples, the input data represents each electrical signal as a sequence of sampled values, wherein each sampled value represents a sample of the electrical signal at the respective electrode at a respective point in time and each sequence is of a same length; and the combined signal comprises a sequence of combined values having a same length as each sequence of sampled values, each combined value corresponding to a respective sampled value from each sequence of sampled values, wherein each corresponding sampled value contributes to the combined value only when one or more characteristics of the sampled value meets one or more predetermined criteria.

In at least one example, the one or more characteristics of the sampled value comprise an amplitude of the sampled value and the one or more predetermined criteria comprises the amplitude exceeding a predetermined value.

In some examples, calculating the value of each combined value may comprise determining a sum of the sampled values that contribute to the combined value. The value of each combined value may be equal to this sum. As another example, the value each combined value may be equal to this sum divided by the number of electrical signals (both contributory and non-contributory).

Thus, in some examples, the value of each combined value is a sum of the sampled values that contribute to the combined value.

Optionally, electrical signal comprises a difference between an electrical response of the respective electrode and an electrical response of a reference electrode. This provides a less noisy electrical signal, taking into account baseline electrical activity (e.g. of the subject). The reference electrode may be positioned on/in the subject for improved noise reduction.

The output data may comprises a waveform representing the combined signal.

There is also proposed a system for aiding a balloon ablation therapy procedure. The system comprises: the processing system herein described; and a control device configured to: communicate with the plurality of electrodes; and control the plurality of electrodes to provide local electric fields and measure the response to the local electric fields.

There is also proposed a computer-implemented method for aiding a balloon ablation therapy procedure that uses an ablation balloon to occlude an anatomical cavity of a subject during the balloon ablation therapy procedure.

The computer-implemented method comprises: receiving input data representing a plurality of electrical signals, each electrical signal being measured by a respective one of a plurality of electrodes disposed in the vicinity of the ablation balloon, wherein each one of the plurality of electrical signals is sensitive to the presence of a dielectric medium injected into the anatomical cavity; processing the plurality of electrical signals contained in the input data to produce a combined signal, wherein each one of the plurality of electrical signals contributes to the combined signal only when one or more characteristics of the electrical signal meets one or more predetermined criteria; and optionally, providing output data comprising the combined signal.

The processing system may be configured to perform any herein described computer-implemented method and vice versa.

There is also proposed a computer program product comprising computer program code means which, when executed on a computing device having a processing system, cause the processing system to perform all of the steps of any herein described method.

### BRIEF DESRIPTION OF THE DRAWINGS

For a better understanding of the invention, and to show more clearly how it may be carried into effect, reference will now be made, by way of example only, to the accompanying drawings, in which:
Figs. 1A and 1B disclose an approach for performing cryoablation with a cryoballoon;
Fig. 2 illustrates an electrophysiological cryoablation system;
Fig. 3 illustrates waveforms of an electrical signal obtained by an EP catheter following injection of a dielectric medium;
Fig. 4 illustrates a processing system according to an embodiment;
Fig. 5 illustrates waveforms of a combined signal following injection of a dielectric medium;
Fig. 6 is a flowchart illustrating a computer-implemented method according to an embodiment; and
Fig. 7 illustrates a processor circuit for use in an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

For example, although the following disclosure may refer to embodiments that include cryoablation procedures, cyroballoons, cryocatheters, RF balloon ablation, or RF balloons, it will be understood that such embodiments are exemplary, and are not intended to limit the scope of the disclosure to those applications. For example, it will be understood that the devices, systems, and methods described herein are applicable to a variety of treatment procedures in which a balloon is used to occlude a body lumen or body cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

As mentioned above, fluoroscopy-based approaches used in guiding balloon ablation procedures, including navigation and deployment of the balloon at the PV ostium and detecting leaks between the PV ostium and the ablation balloon suffer from various drawbacks. It may be desirable to provide an approach for guiding a full ablation procedure without using fluoroscopy and/or contrast agent. The present disclosure provides systems, methods, and devices for guiding a balloon ablation procedure using EP imaging and EP- based leak detection techniques. As will be further described below, an electrophysiology system can be used to provide for image-guided delivery of a balloon ablation device, such as a cryoballoon or RF balloon, into a body cavity, such as the left atrium, and to facilitate a fully-occluding placement of the cryoballoon at an ablation site.

A region of interest of a subject can comprise an anatomical cavity of the subject such as a chamber of a heart and or a part of a vessel (artery or vein) connected to such chamber.

The present disclosure proposes a mechanism for aiding a balloon ablation therapy procedure, and in particular, to provide information useful for establishing how successfully a balloon has occluded an anatomical cavity. A plurality of electrodes (positioned upstream or distally of the ablation balloon) each provide a signal responsive to the presence and/or absence of a dielectric medium injected into the anatomical cavity. A combined signal is generated, to which the signals provided by the electrodes only contribute if they meet some predetermined criterion/criteria.

The present invention recognizes that a combined signal would provide useful clinical information for establishing a success of an occlusion. However, simply combining all electrical signals would result in a combined signal with an extremely low signal to noise ratio. By only allowing signals to contribute to the combined signal when they meet some predetermined criteria, a significant improvement to a signal to noise ration can be achieved.

Embodiments may be employed in any suitable balloon ablation therapy procedure.

Embodiments make use of a dielectric medium that changes the dielectric properties of material into which it is injected. A dielectric medium may be otherwise labelled dielectrically modifying fluid during the occlusions measurements. Advantageously, the medium does not need to be an X-ray contrast agent (Fluoroscopic dye), but may be a less harmful and less viscous saline or dextrose-in-water (D5W) fluid. This shortens the required test time and enables a zero-fluoroscopy (zero-F) and zero-dye (zero-D) procedure. The medium used in embodiments will herein after be referred to as dielectric medium in the sense that it is a medium that has dielectric properties different from that of normal blood. An example of such property may be electrical conductivity which for normal blood is ∼6.6 mS/cm. The dielectric medium may thus have an electrical conductivity different form that of normal blood. One working example of a dielectric medium is Fluoroscopic dye Omnipaque^{™} marketed by General Electric^{™}. Another non-ionic medium may comprise glucose also known as dextrose which also is a non-ionic compound. A 5 weight % solution in water (D5W) has a conductivity of around 0.01 mS/cm. The above does not mean that agents with higher conductivity than blood cannot be used with the method and system as described herein. For example, 0.9% saline is known to be a medium that has a higher conductivity (-14.5 mS/cm) than blood and it may be used with the methods and systems disclosed herein.

Embodiments also make use of signals generated by an electrophysical (EP) catheter, which (in use) is positioned distally to an ablation balloon in an anatomical cavity, e.g. downstream. There are a plurality of electrodes on the electrophysical catheter, each of which generates an electrical signal responsive to the presence of a dielectric medium injected into the anatomical cavity. For instance, each electrical signals may be responsive to dielectric properties of material surrounding the electrode.

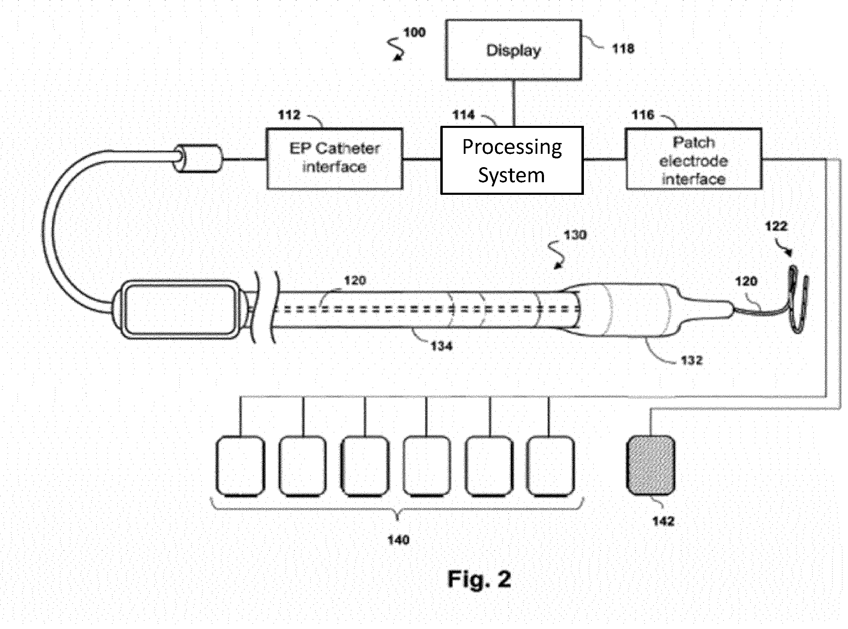

For the sake of improved contextual understanding, Fig. 2 provides a diagrammatic schematic view of an EP cryoablation system 100, for use with embodiments of the present disclosure. The skilled person would appreciate how other ablation system could be used to a similar effect.

The EP cryoablation system 100 includes an EP catheter 120. In some embodiments, the EP catheter 120 extends through a lumen of a cryoballoon catheter 130. The EP catheter 120 is communicatively coupled to an EP catheter interface 112, which is communicatively coupled to a processing system 114.

The cryoballoon catheter 130 may include an ablation balloon 132 (here: an inflatable cryoballoon) 132 coupled to a distal portion of a flexible elongate member 134, which may comprise a sheath. The EP catheter 120 may be positioned at least partially within a lumen of the flexible elongate member 134 such that a distal portion 122 of the EP catheter 120, which comprises a plurality of electrodes positioned on an elongate tip member, may protrude out a distal end of the cryoballoon catheter 130. In this way, the electrodes are positioned distally of the ablation balloon 132,

In one example, the cryoballoon catheter 130 comprises a lumen configured to slidably receive the EP catheter 120. The EP catheter 120 may comprise a flexible elongate member configured to be positioned within the cryoballoon catheter 130. This facilitates introduction of the cryoballoon catheter 130 into the anatomical cavity first and for the EP catheter 120 to be advanced distally within the cryoballoon catheter 130 until the distal portion 122, and therefore the electrodes, of the EP catheter 120 protrude(s) out the distal end of the cryoballoon catheter 130 at a region of interest (e.g., an ablation site).

As previously mentioned, the distal portion 122 of the EP catheter comprises a plurality of electrodes positioned on the elongate tip member ("EP catheter electrodes"). In some embodiments, the EP catheter comprises between 8 and 10 electrodes. However, the EP catheter can include other numbers of electrodes, including 2, 4, 6, 14, 20, 30, 60, or any other suitable number of electrodes, both larger and smaller. Electrical signals generated by the catheter are passed to the processing system 114, via the EP catheter interface 112.

The elongate tip member may thereby be configured to be positioned distally of the cryoballoon. The elongate tip member may be biased, shaped, or otherwise structurally configured to assume a shape, such as a circular shape, in which the electrodes are spaced from one another about one or more planes. For example, the EP catheter can be a spiral mapping catheter (SMC) in which electrodes are distributed along an elongate tip member in a spiral configuration, e.g. with a 15mm, 20mm or 25mm loop diameter. In this way, the plurality of electrodes can (in use) be arranged circularly in the anatomical cavity.

In some embodiments, commercially-available EP catheters can be used with the system 100, including the Achieve^{™} and Achieve Advance^{™} Mapping catheters manufactured by Medtronic^{™}. The EP catheter can be designed for use with the Arctic Front^{™} Family of Cardiac Cryoablation Catheters and/or the FlexCath^{™} Advance Steerable Sheath, manufactured by Medtronic^{™}.

In some embodiments, the cryoballoon 132 comprises a plurality of electrodes positioned on an exterior surface of the cryoballoon 132 and configured to obtain data used to determine occlusion at an ablation site. Further details regarding EP catheters and assemblies can be found in, for example, U.S. Patent No. 6,002,955, titled "Stabilized Electrophysiology Catheter and Method for Use," the entirety of which is hereby incorporated by reference.

The skilled person would be able to replace the EP catheter 120 and the cryoablation catheter 130 with any other combination of EP catheter and ablation catheter (e.g. a radiofrequency ablation catheter).

Proposed embodiments make use of electrical signals generated by EP catheter electrodes. Each electrical signal is responsive to the presence or absence of a dielectric medium in the vicinity of the EP catheter electrode. For instance, an electrical signal may be a voltage signal induced (in an EP catheter electrode) by an electric field. The value of the voltage signal will change responsive to changes in the dielectric properties of surrounding material. A dielectric medium is any suitable fluid (e.g. dye) that changes the dielectric properties of material (e.g. blood) into which it is injected. The electric field may be generated by other EP catheter electrode(s) and/or external (to the body) electrodes, such as body patch electrodes. The voltage signal may represent a voltage difference between the EP catheter electrode and a reference electrode (e.g. a reference body patch electrode).

For the exemplary system 100, external body patch electrodes are used to generate the electric fields (to which the EP catheter electrodes respond). Thus, the system 100 further comprises a plurality of body patch electrodes 140 and a reference patch electrode 142 communicatively coupled to a patch electrode interface 116, which is in communication with the processing system 114. For example, the patch electrodes 140 and the reference electrode 142 may be coupled to the patch electrode interface 116 via electrical cables. In the embodiment shown in Fig. 2, the system 100 comprises six external body patch electrodes 140 and one reference electrode 142. However, in some embodiments, the system 100 comprises fewer or more body patch electrodes 140 than are shown in Fig. 2, including 1, 2, 4, 8, 19, 12, 20, or any other suitable number of body patch electrodes, both lager and smaller. Further, in some embodiments, the system 100 may include more than one reference electrode 142, including 2, 4, 5, or any other suitable number of reference patch electrodes.

The patch electrodes 140 and reference electrode 142 is usable (together with the electrodes of the EP catheter) for a variety of purposes. In particular, and as previously described, the patch electrodes 140 may be controlled to generate electrical fields. The response, e.g. the voltage levels, of the electrodes of the EP catheter to these electrical fields (e.g. relative to the response of the reference electrode 142) is dependent upon the position of the electrodes in the catheter and dielectric properties of surrounding material.

One known use for the patch, reference and EP catheter electrodes include the mapping of the anatomical cavity and the identification of the relative location of the EP catheter. A more detailed explanation of the use of body patch electrodes and catheter electrodes to map body volumes and visualize the locations of EP catheters within the map can be found in, for example, U.S. Patent No. 10,278,616, titled "Systems and Methods for Tracking an Intrabody Catheter," and U.S. Patent No. 5,983,126, titled "Catheter Location System and Method," the entireties of which are hereby incorporated by reference as well as in publications WO2018130974 and WO2019034944 of the corresponding international patent applications the entireties of which are herein incorporated by reference.

The present disclosure makes use of the electric fields to generate electrical signals (at the EP catheter) responsive to dielectric medium injected into the anatomical cavity. The electrical signals may be a voltage difference between the EP catheter electrode and the reference patch electrode. The injection of dielectric medium changes the dielectric property of material surrounding the electrodes, which is reflected in the signals generated by the EP catheter electrodes, e.g. the measured voltage changes. This property can be used to monitor a success of occlusion, as a failure of the signals to change after a dielectric medium is injected (or a return to the original pre-injection signals) indicates that the occlusion is not successful and/or complete.

Each EP catheter electrode will provide its own (e.g. voltage) signal, so that the electrical response of each EP catheter electrode to an injected dielectric medium represents a success of occlusion at the location of said EP catheter electrode.

A more complete (and alternative) description of how to generate electrical signals responsive to the presence and/or absence of a dielectric medium is disclosed by WO2021001338, the entirety of which is incorporated herein by reference.

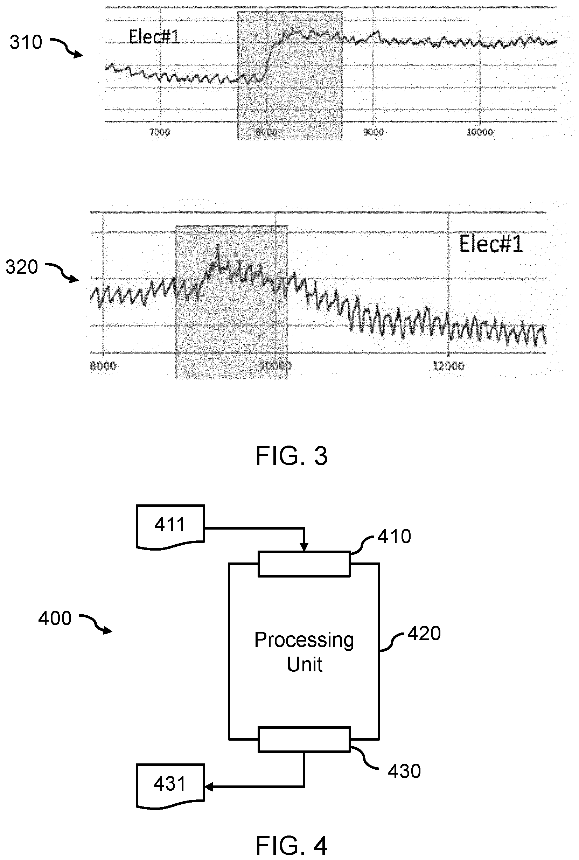

Fig. 3 illustrates examples of electrical signals (i.e. not a combined signal) generated by an EP catheter electrode Elec#1 in different circumstances.

A first waveform 310 illustrates the voltage response of an electrode on an EP catheter to the injection of a dielectric medium into the anatomical cavity when the anatomical cavity has been successfully blocked by an ablation balloon positioned less distally (i.e. downstream of blood flow) to the electrode.

In the first waveform 310, the voltage increases (due to the dielectric properties changing), and then remains substantially constant. This indicates that there is no/negligible leakage of the dielectric medium past the ablation balloon via the EP catheter electrode.

A second waveform 320 illustrates the voltage response of an electrode on an EP catheter to the injection of a dielectric medium into the anatomical cavity when the anatomical cavity has not been successfully blocked by an ablation balloon positioned less distally (i.e. downstream of blood flow) to the electrode.

In the second waveform 320, the voltage increases (due to the dielectric properties changing), and then reduces to pre-injection level. This indicates that there is leakage of the dielectric medium past the ablation balloon via the EP catheter electrode, i.e. the balloon has not successfully occluded the anatomical cavity.

It will be appreciated that the electrical signal of each EP catheter electrode, such as those illustrated in Fig. 3, are time dependent.

Embodiments of the present disclosure recognize that the electrical signals are highly sensitive to noise, such as that caused by slight movements of the EP catheter, movement of the subject (e.g. as a result of respiration) and/or electrical activity of the subject. There is therefore a desire to improve the signal to noise ratio of signals representative of occlusion success.

Embodiments of this disclosure propose the use of a combined signal, to which electrical signals of different EP catheter electrodes are able to contribute. In particular, the electrical signals of different EP catheter electrodes may only be able to contribute to the combined signal if they meet one or more predetermined criteria. The combined signal can thereby be reflective of the occlusion state of the balloon with a high degree of accuracy.

In particular, the combined signal can provide additional useful information on the success of the occlusion and, if present, properties or characteristics of any leak.

The generation of the combined signal is performed by a processing system, such as the processing system 114. It will be appreciated that the operation of the processing system in generating the combined signal can be performed independently of the operation of obtaining the electrical signals. For instance, the electrical signals can be stored for later processing by the processing system.

It should also be recognized that the function and advantages provided by the processing system is not solely reliant upon the manner in which the electrical signals are obtained, but rather relies upon further processing of the obtained signals in order to achieve its underlying advantages. More particularly, the proposed approach defines the mode of operation of a technical device (i.e. the processing system), namely how to generate useful clinical data for identifying occlusion information with the aid of information generated by the electrodes. This process does not directly cause any effects on the human body. There is therefore no processing system induced therapeutic effect on the body that could be functionally related to the steps performed by the processing system in generating the combined signal.

Embodiments in which the plurality of EP catheter electrodes are arranged circularly in the anatomical cavity are particularly advantageous, as it allows the tracking of leakages and/or occlusions around the boundary of the anatomical cavity. Thus, the combined signal may be generated from electrical signals obtained from electrodes that monitor different circumferential locations in the anatomical (e.g. resulting from the circular shape of the EP catheter during the procedure).

The system 100 may comprise a display device. The display device may be configured to provide a visual representation of information generated by the processing system (e.g. of the combined signal).

For completeness, an outline will be presented of an imaging function, which is able to generate a volume representation of the anatomical cavity and/or identify the location of the EP catheter. Reference will be made to the system illustrated in Fig. 2, for improved contextual understanding.

The electrodes of the EP catheter may be labelled "internal electrodes" and the patch electrodes may be labelled "external electrodes". A distance between each of the internal electrodes (inter-electrode spacing) and their corresponding electrical weight lengths are predetermined and hence known.

The mapping guidance system 114 is configured to provide and receive signals from the electrodes to perform a cardiac dielectric imaging process. It provides 3D electro- anatomical visualization for guiding catheter-based treatment of cardiac arrhythmias. It utilizes wide-band dielectric sensing and a technology based on the bending of electric fields to generate high-resolution 'CT-like' full 3D images as well as flattened 3D panoramic views of the cardiac anatomy. Sensor-less diagnostic and ablation EP catheters are prequalified to operate with the system.

The system generates a global low intra-body electrical field by the set of external sensors, which are differentially excited, together with a local electrical field via the internal electrodes on the indwelling catheter. The internal and external electrodes are all both emitters and receivers in the frequency range of 20 kHz - 100 kHz. A reference patch electrode serves as an electric reference for all voltage measurements (creating a V-space).

The distribution of the induced electric field is inherently inhomogeneous due to the different dielectric properties and absorption rates (related to conductivity) of the interrogated tissues. The external electrodes measure the global general effects and distorted electric field whereas the internal electrodes measure the local effect and tissue response.

Throughout the imaging process, the imaged volume continuously grows at the sampling rate of 100 Hz. An optimal transfer function is used to transform the voltages to Euclidian coordinates (creating an R-space) while maintaining the known pre-qualified catheter characteristics (electrode spacing and electrical weight length, functioning as an internal ruler) as well as a set of other constraints. This transfer function is repeatedly defined and applied globally.

Using the updated R-space cloud of points a reconstruction algorithm generates a 3D image. Regions with inherently marked steep gradients in the electric field (i.e. drainage of vessels into or out of a cardiac chamber as well as the A-V and V-A valves) are picked up uniquely by the system and imaged even without physically visiting them with the catheter. The system thus can image at locations beyond the catheter.

The continuous combined global and local field measurements enable sophisticated continuous detection and effective handling of inconsistencies and outliers, level of electrode coverage (by measuring inter-correlations), pacing (saturation), as well as physiological drift.

Drift is detected by applying a moving window over time and applying continuous correction whereby the catheter location remains accurate throughout the whole procedure making the system resilient to drift. Cardiac and respiratory motion are also compensated.

For imaging, at least two electrodes on a catheter/wire/pacing lead are necessary, whereas even a single electrode can be spatially localized after the initial obligatory computation of the R-field. The system has been proposed for guiding CryoBalloon (CB) Pulmonary Vein Isolation (PVI) procedures while potentially minimizing exposure to fluoroscopy and obviating dye injection.

In the diagram shown in Fig. 2, the EP catheter interface 112, processing system 114, and patch electrode interface 116 are illustrated as separate components. However, in some embodiments, the interfaces 112, 116 and the processing system 114 may be components of a single console or computing device with a single housing. In other embodiments, the interfaces 112, 116 and the processing system 114 may comprise separate hardware components (e.g., with separate housings) communicatively coupled to one another by electrical cables, wireless communication devices, fiber optics, or any other suitable means of communication. Further, in some embodiments, the EP catheter interface 112 may also function as an interface for the cryoballoon catheter 130 to control inflation of the cryoballoon 132, cooling/heating of the cryoballoon 132, etc. In other embodiments, the cryoballoon catheter 130 is controlled by a separate interface or control system, such as a console.

The processing system 114 may be coupled to a display device 118, which may be configured to provide visualizations of a cryoablation procedure to a physician. For example, the processing system 114 may be configured to generate EP images of the body cavity, visualizations of the propagation of EP waves across the tissue of the body cavity, indications of occlusion by the cryoballoon at an ablation site, or any other suitable visualization. These visualizations may then be output by the processing system 114 to the display device 118.

Fig. 4 illustrates a processing system 400 according to an embodiment. The processing system 400 may, for instance, represent the processing system 114 of Fig. 2.

The processing system comprises an input interface 410. The input interface is configured to receive input data 411 representing a plurality of electrical signals. Each electrical signal is measured by a respective one of a plurality of electrodes disposed on an elongate tip member of an electrophysiological catheter (i.e. EP catheter electrodes). Each electrode is positioned distally of the ablation balloon in the anatomical cavity and each electrical signal is sensitive to the presence of a dielectric medium injected into the anatomical cavity.

The processing system 400 also comprises a processing unit 420 communicatively coupled to the input interface and configured to process the electrical signals contained in the input data to produce a combined signal. Each electrical signal contributes to the combined signal only when one or more characteristics of the electrical signal meets one or more predetermined criteria.

The combined signal thereby represents information on an overall occlusion success (and carries information on the overall occlusion) of the anatomical cavity by the ablation balloon.

The processing system 400 may comprise an output interface 430 communicatively coupled to the processing unit and configured to provide output data 431 comprising the combined signal.

The processing unit 420 may be configured such that an electric signal only contributes to the combined if/when it meets a predetermined criterion or criteria. For instance, an electrical signal may only be able to contribute to the combined signal if an amplitude/magnitude of the electrical signal exceeds some predetermined threshold, or a gradient of the electrical signal exceeds some predetermined threshold.

Limiting the contribution to only those electrical signals having an amplitude/magnitude that exceeds a predetermined threshold improves the signal to noise ratio of the combined signal, and ensures that only those signals that indicate an occlusion contribute to the combined signal, improving a relevance and accuracy of the combined signal.

Limiting the contribution to only those electrical signals having a gradient that exceeds a predetermined threshold means the combined signal will provide useful information on changes in the dielectric characteristics of the anatomical cavity, allowing leakages or injections to be more readily identified.

The processing unit may generate the combined signal by summing or averaging the electrical signals that contribute to the combined signal. In one example, the contributing electrical signals are summed, and the end result is divided by the total number of (both contributing and non-contributing) electrical signals.

The processing unit may, for instance, repeatedly perform this process at different points in time, so that an electrical signal that does not meet the predetermined criteria at a first point in time will not contribute to the combined signal at the first point in time, but if it meets the criteria at a second point in time, then it will contribute.

Put another way, each electrical signal and the combined signal may be time dependent; and each electrical signal may only contribute to the combined signal, at a particular point in time for the combined signal, when one or more characteristics of the electrical signal at the particular point in time meets one or more predetermined criteria. Thus, the contribution from different electrical signals to the combined signal may change overtime.

One approach to implement this concept is for the input data to represent each electrical signal as a sequence of sampled values, wherein each sampled value represents a sample of the electrical signal at the respective electrode at a respective point in time and each sequence is of a same length. The combined signal may then comprise a sequence of combined values having a same length as each sequence of sampled values, each combined value corresponding to a respective sampled value from each sequence of sampled values, wherein each corresponding sampled value contributes to the combined value only when one or more characteristics of the sampled value meets one or more predetermined criteria.

The output data, provided by the optional output interface, may be or comprise a waveform that represents the combined signal. This provides a simple and intuitive approach for visualizing the combined signal.

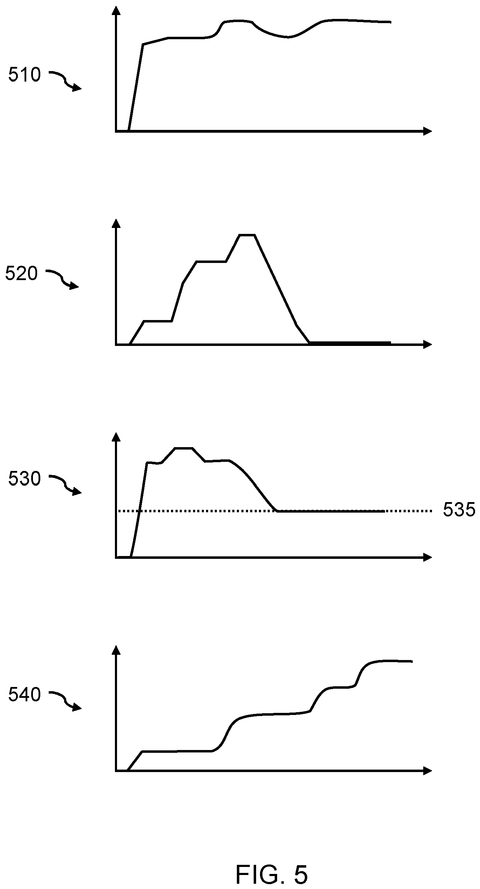

Fig. 5 illustrates various waveforms for a combined signal.

For this example, electrical signals only contribute to the combined signal when an amplitude/magnitude of the electrical signal exceeds a predetermined threshold. The contributing electrical signals are summed to produce the combined signal. This provides a combined signal with an improved signal-to-noise ratio.

A first waveform 510 illustrates a scenario in which the ablation balloon successfully occludes the anatomical cavity (i.e. the entire way around). The combined signal thereby increases and remains substantially constant thereafter, indicating that there is no leakage (i.e. there is no reduction in the combined signal).

A second waveform 520 illustrates a scenario in which there is significant leakage past the ablation balloon. There is an increase in the value of the combined signal, before a sudden drop back down to pre-injection levels.

A third waveform 530 illustrates a scenario in which there is a small leak past the ablation balloon. There is an increase in the value of the combined signal, before this value decreases slightly to a new baseline level 535. This is caused by only part of the anatomical cavity being occluded, so that there is no blood flow past part of the ablation balloon. This means that some of the dielectric medium is maintained in the vicinity of some of the electrodes.

A fourth waveform 540 illustrates a scenario in which there is a slow or late injection of dielectric medium into the anatomical cavity. The rise of the combined signal is slow (but other forms of slow/late injection may instead exhibit a sudden, late rise).

The examples above demonstrate how a single combined signal is able to provide more useful information than a single electrical signal (of a single electrode). The use of multiple electrical signals that variably contribute or do not contribute to the combined signal thereby provide a clinically useful combined signal that

A processing system or method according to an embodiment may be configured to provide, at a user interface, a user-perceptible output (such as a visual representation or the like) of the combined signal.

It will be appreciated that other forms of information derived from the electrical signals may also be included in the user-perceptible output.

For instance, the electrical signal from each electrode may be processed and used to assess whether there is a predicted leakage past that electrode. This information may be presented to the subject.

As another example, it has previously been described how electrical signals could be processed to identify a location of the electrode and/or to map the anatomical cavity. In these examples, a representation of the position of each electrode and/or the mapped anatomical cavity may be presented to the user.

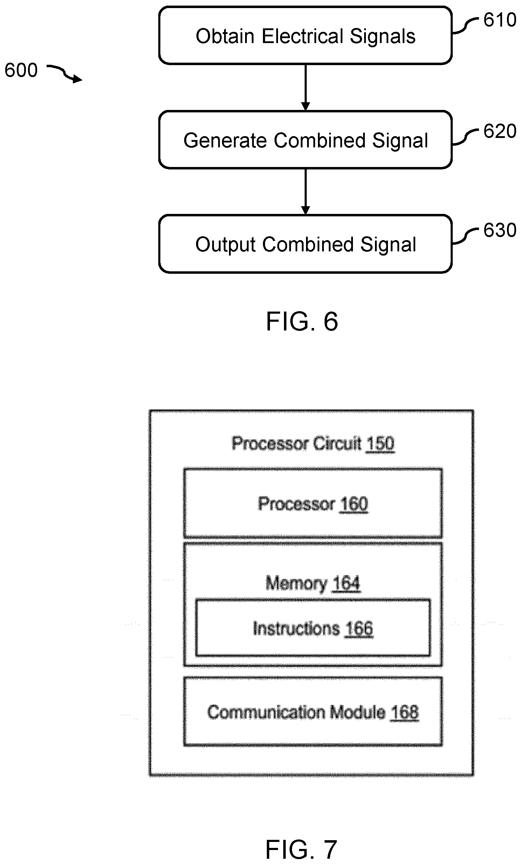

Fig. 6 illustrates a computer-implemented method according to an embodiment. The computer-implemented method may be carried out by the processing system (e.g. the processing system 400 of Fig. 4).

The computer-implemented method is configured for aiding a balloon ablation therapy procedure that uses an ablation balloon to occlude an anatomical cavity of a subject during the balloon ablation therapy procedure.

The computer-implemented method 600 comprises receiving 610 input data representing a plurality of electrical signals, each electrical signal being measured by a respective one of a plurality of electrodes disposed on an elongate tip member of an electrophysiological catheter, wherein each electrode is positioned distally of the ablation balloon in the anatomical cavity and each electrical signal is sensitive to the presence of a dielectric medium injected into the anatomical cavity.

The method 600 also comprises processing 620 the electrical signals contained in the input data to produce a combined signal, wherein each electrical signal contributes to the combined signal only when one or more characteristics of the electrical signal meets one or more predetermined criteria.

The computer-implemented method may comprise a step 630 providing output data comprising the combined signal. The output data may comprise a waveform representing the combined signal.

Step 630 may comprise controlling a user interface to provide a user-perceptible output of the combined signal, e.g. in the form of a visual representation. In other examples, the output data is provided to a further processing system for assessing and/or analyzing the output data (e.g. for assessing an occlusion success or failure of the ablation balloon).

Fig. 7 is a schematic diagram of a processor circuit 150, according to embodiments of the present disclosure.

The processor circuit 150 may be implemented in any processing system and/or processing unit herein described. The processor circuit 150 can carry out one or more steps described herein. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non volatile memory, or a combination of different types of memory. In an embodiment, the memory 164 includes computer-readable medium, which may be a non-transitory computer- readable medium. The computer-readable medium may store instructions. For example, the memory 164, or computer-readable medium may have program code recorded thereon, the program code including instructions for causing the processor circuit 150, or one or more components of the processor circuit 150, to perform the operations described herein. For example, the processor circuit 150 can execute operations described with reference to Fig. 6 , including the operations described with reference to the methods 200, 400.

Instructions 166 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 164, with the code recorded thereon, may be referred to as a computer program product. The communication module 168 can include any electronic circuitry and/or logic circuitry to facilitate direct or indirect communication of data between the processor circuit 150, the mapping and guidance system 114, the EP catheter 120, the cryoballoon catheter 130, and/or the display 118. In that regard, the communication module 168 can be an input/output (I/O) device. In some instances, the communication module 168 facilitates direct or indirect communication between various elements of the processor circuit 150 and/or the system 100 (Fig. 2). In some embodiments, the processor circuit 150 may further comprise an electrical signal generator and/or an electrical signal measurer configured to control the electrodes of the EP catheter to emit and/or detect electrical signals, including voltages, impedances, and currents.

In one embodiment, the device as defined herein comprises or is a computing device or a computer whether mobile or stationary. Such computing device may take the form of a workstation.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (114, 400) for aiding a balloon ablation therapy procedure that uses an ablation balloon (30, 132) to occlude an anatomical cavity (14) of a subject during the balloon ablation therapy procedure, the processing system comprising:
an input interface (410) configured to receive (610) input data (411) representing a plurality of electrical signals (310, 320) obtained from a plurality of electrodes disposed in the vicinity of the ablation balloon, wherein each one of the plurality of electrical signals is sensitive to the presence of a dielectric medium injected into the anatomical cavity;
a processing unit (420) communicatively coupled to the input interface and configured to process (620) the plurality of electrical signals contained in the input data to produce a combined signal (510, 520, 530, 540), wherein each one of the plurality of electrical signals contributes to the combined signal only when one or more characteristics of said electrical signal meets one or more predetermined criteria; and
optionally, an output interface (430) communicatively coupled to the processing unit and configured to provide (630) output data (431) comprising the combined signal.

2. The processing system of claim 1, wherein the one or more characteristics of the electrical signal comprise an amplitude of the electrical signal and the one or more predetermined criteria comprise the amplitude of the electrical signal breaching a first predetermined threshold.

3. The processing system of claim 1 or 2, wherein the plurality of electrodes are arranged around a hypothetical closed shape in the anatomical cavity.

4. The processing system of any of claims 1 to 3, wherein the plurality of electrodes are positioned distally of the ablation balloon in the anatomical cavity and/or upstream of a blood flow direction through the anatomical cavity.

5. The processing system of any of claims 1 to 4, wherein the one or more characteristics of the electrical signal comprise a gradient of the amplitude of the electrical signal and the one or more predetermined criteria comprises the gradient of the amplitude of the electrical signal breaching a second predetermined threshold.

6. The processing system of any of claims 1 to 5, wherein:
each electrical signal and the combined signal is time dependent; and
each electrical signal only contributes to the combined signal, at a particular point in time for the combined signal, when one or more characteristics of the electrical signal at the particular point in time meets one or more predetermined criteria.

7. The processing system of any of claims 1 to 6, wherein the combined signal comprises a sum of the electrical signals that contribute to the combined signal.

8. The processing system of any of claims 1 to 7, wherein:
the input data represents each electrical signal as a sequence of sampled values, wherein each sampled value represents a sample of the electrical signal at the respective electrode at a respective point in time and each sequence is of a same length; and
the combined signal comprises a sequence of combined values having a same length as each sequence of sampled values, each combined value corresponding to a respective sampled value from each sequence of sampled values, wherein each corresponding sampled value contributes to the combined value only when one or more characteristics of the sampled value meets one or more predetermined criteria.

9. The processing system of claim 8, wherein the one or more characteristics of the sampled value comprise an amplitude of the sampled value and the one or more predetermined criteria comprises the amplitude exceeding a predetermined value.

10. The processing system of any of claims 8 or 9, wherein the value of each combined value is a sum of the sampled values that contribute to the combined value.

11. The processing system of any of claims 1 to 10, wherein each electrical signal comprises a difference between an electrical response of the respective electrode and an electrical response of a reference electrode.

12. The processing system of any of claims 1 to 11, wherein the output data comprises a waveform representing the combined signal.

13. A system for aiding a balloon ablation therapy procedure, the system comprising:
the processing system of any of claims 1 to 13; and
a control device configured to:
communicate with the plurality of electrodes; and
control the plurality of electrodes to provide local electric fields and measure the response to the local electric fields.

14. A computer-implemented method (600) for aiding a balloon ablation therapy procedure that uses an ablation balloon (30, 132) to occlude an anatomical cavity (10) of a subject during the balloon ablation therapy procedure, the computer-implemented method comprising:
receiving (610) input data (411) representing a plurality of electrical signals (310, 320), each electrical signal being measured by a respective one of a plurality of electrodes disposed in the vicinity of the ablation balloon, wherein each one of the plurality of electrical signals is sensitive to the presence of a dielectric medium injected into the anatomical cavity;
processing (620) the plurality of electrical signals contained in the input data to produce a combined signal (510, 520, 530, 540), wherein each one of the plurality of the electrical signals contributes to the combined signal only when one or more characteristics of said electrical signal meets one or more predetermined criteria; and
optionally, providing (630) output data (431) comprising the combined signal.

15. A computer program product comprising computer program code means which, when executed on a computing device having a processing system, cause the processing system to perform all of the steps of the method according to claim 14.
